# EUROPEAN PATENT SPECIFICATION

(11) **EP 2 155 237 B1**
(45) Date of publication and mention of the grant of the patent: **10.02.2016**
(21) Application number: 08761679.3
(22) Date of filing: 20.05.2008
(51) Int. Cl.: A61K 38/50, A61P 35/00, A61P 29/00, C12N 9/82

(54) **GLYCOSYLASPARAGINASE FOR TREATMENT OF CANCER**
GLYKOSYLASPARAGINASE ZUR BEHANDLUNG VON KREBS
GLYCOSYLASPARAGINASE POUR LE TRAITEMENT DE CANCERS

(30) Priority: 08.06.2007 FI 20070455; 08.06.2007 US 942721 P
(43) Date of publication of application: 24.02.2010
(73) Proprietor: Oy Reagena Ltd, 70900 Toivala (FI)
(72) Inventor: MONONEN, Ilkka, FI-20700 Turku (FI); KELO, Eira, FI-70910 Vuorela (FI)
(74) Representative: Öhman, Ann-Marie
(86) International application number: PCT/FI2008/050283
(87) International publication number: WO 2008/148932

(56) References cited:
- EP-A- 0 811 687
- WO-A-03/038113
- US-A- 4 617 271
- US-A1- 2004 197 299
- US-B1- 6 251 388
- YLIKANGAS PAIVI ET AL: "Serious neutropenia in ALL patients treated with L-asparaginase may be avoided by therapeutic monitoring of the enzyme activity in the circulation" THERAPEUTIC DRUG MONITORING, vol. 24, no. 4, August 2002 (2002-08), pages 502-506, XP009109534 ISSN: 0163-4356
- REIFF A ET AL: "Treatment of collagen induced arthritis in DBA/1 mice with L-asparaginase." CLINICAL AND EXPERIMENTAL RHEUMATOLOGY 2001 NOV-DEC, vol. 19, no. 6, November 2001 (2001-11), pages 639-646, XP009109566 ISSN: 0392-856X
- KAARTINEN V ET AL: "SUBSTRATE SPECIFICITY AND REACTION MECHANISM OF HUMAN GLYCOASPARAGINASE THE N-GLYCOSIDIC LINKAGE OF VARIOUS GLYCOASPARAGINES IS CLEAVED THROUGH A REACTION MECHANISM SIMILAR TO L ASPARAGINASE" JOURNAL OF BIOLOGICAL CHEMISTRY, vol. 267, no. 10, 1992, pages 6855-6858, XP002506519 ISSN: 0021-9258 cited in the application
- Dominika Borek ET AL: "Expression, purification and catalytic activity of Lupinus luteus asparagine [beta]-amidohydrolase and its Escherichia coli homolog", European Journal of Biochemistry, vol. 271, no. 15, 14 July 2004 (2004-07-14), pages 3215-3226, XP055188258, GB ISSN: 0014-2956, DOI: 10.1111/j.1432-1033.2004.04254.x

## Description

### FIELD OF THE INVENTION

This invention relates to the use of a glycosylasparaginase polypeptide with L-asparaginase activity or its precursor in the treatment or prevention of cancers. The invention concerns also a novel pharmaceutical composition.

### BACKGROUND OF THE INVENTION

L-asparagine is an amino acid essential for the growth of cells including cancer cells such as leukemic cells. In normal cells asparagine is formed from aspartic acid or aspartates with asparagine synthetase, an asparagine-synthesizing enzyme, while cancer cells, especially leukemic cells are deficient in the asparagine-synthesizing activity.

L-Asparaginase (ASNase; EC 3.5.1.1), which catalyzes the deamination of L-asparagine (Asn) to L-aspartic acid (Asp) and ammonia, is widely used in the treatment of acute lymphoblastic leukemia (ALL) in combination with other anti-cancer drugs^{1,2}. Earlier studies have suggested that L-asparaginase might be effective in the treatment of some subtypes of acute myeloid leukaemia, natural killer (NK)-cell tumors³¹, multiple myeloma (Agrawal NR et al., 2003, Cancer, 98, 94-99), solid tumors (Taylor CW et al., 2001, Cancer Chemother Pharmacol, 47, 83-88) and certain non-cancerous diseases such as rheumatoid arthritis (Reiff A et al., 2001, Clin Exp Rheumatol, 19, 639-646) as well. The antitumour activity of ASNase is based on the fact that certain tumour cells, which lack L-asparagine synthetase activity, are dependent on their external supply of Asn^{3,4}. Depletion of this supply by L-asparaginase induces apoptosis in tumour or leukemia cell lines *in vitro*^{9,10}. Normal cells are not be damaged because of their ability to synthesize L-asparaginase.

Clinically used ASNases have mainly been isolated from bacterial sources, namely *Escherichia coli* and *Erwinia carotovora* (also known as *Erwinia chrysanthemi*)²*.* The major causes of the various side effects during the L-asparaginase treatment have been related either to the L-glutaminase activity of L-asparaginasesl^{11,12} or the development of antibodies against the bacterial proteins foreign to human body^{13,14,15}. The decline of the plasma L-glutamine levels during the L-asparaginase treatment affects L-glutamine metabolism and nitrogen transportation in blood thus causing disorders especially in liver^{11,12}. Another problem of bacterial ASNases is their rapid clearance from the systemic circulation, which makes frequent injections of the drug necessary. *E. coli* L-asparaginase has been linked to polyethylene glycol (PEG-asparaginase) in order to reduce the side effects of the L-asparaginase treatment and prolong the half-life of the enzyme during the therapy¹⁶.

As examples of commercialized L-asparaginase preparations can be mentioned Crasnitin® and Elspar® (enzyme obtained from *Escherichia coli),* Erwinase® (enzyme obtained from *Erwinia chrysanthemi*) and Oncospar® (PEG-linked L-asparaginase).

Glycosylasparaginase (GA; aspartylglucosaminidase; EC 3.5.1.26) is an enzyme which is present in the human body. Certain individuals may, however, due to a genetic defect suffer from GA-deficiency. As a result thereof, an aspartylglucosamine complex (comprising of asparagine and sugar) is formed. Accumulation of aspartylglucosamine in tissues and body fluids will in turn lead to a serious mental disability, aspartylglucosaminuria (AGU) for the individual.

Glycosylasparaginase hydrolyzes the N-glycosidic carbohydrate-to-protein linkage region, aspartylglucosamine, to L-aspartic acid and 1-amino-N-acetylglucosamine¹⁷ through a reaction mechanism similar to L-asparaginase¹⁸. At least human GA, but probably GA enzymes isolated from other sources or modified GA possesses L-asparaginase activity by catalyzing the hydrolysis of L-asparagine to L-aspartic acid and ammonia¹⁹, but in contrast to bacterial L-asparaginases it lacks L-glutaminase activity¹⁸. Glycosylasparaginase is a glycoprotein that is normally transported into lysosomes via the mannose-6-phosphate-receptor-dependent transport system and the dephosphorylation of its carbohydrate chains reduces its transportation into lysosomes of EBV-transformed lymphoblasts by ca 90%²⁰ without affecting the enzyme activity.

Although human GA is known to possess L-asparaginase activity by catalyzing the hydrolysis of L-asparagine to L-aspartic acid and ammonia, and thus would be a well tolerable alternative for bacterial L-asparaginases for the treatment of cancers, nobody has so far suggested this use.

The published patent application US 2004/0197299 describes the use hydrolase polypeptides, particularly glycosylasparaginases, including fragments, analogues and modifications thereof as well as their precursors for use in topically applicable cosmetic/dermatological preparations, especially for treating dry skin and combating skin desquamation.

The published patent application US 2005/0124593 discloses that the expression of the 25943 gene, a gene encoding glycosylasparaginase, is upregulated in certain tumors. Thus, inhibition of this gene activity is suggested as a method for treating cancers.

The publication S Virta et al., J Gene Med 2006; 8:699-706 suggests a virus-mediated gene therapy for treatment of aspartagylglucosaminuria (AGU) by promoting the expression of the glucosylasparaginase gene.

The published European patent application EP 0811687A2 discloses polypeptides having L-asparaginase activity for treatment of tumours. The polypeptides described have however, amino acid sequences different from that of glucosylasparaginase.

### SUMMARY OF THE INVENTION

The present invention is based on a study, where the inventors show that human glycosylasparaginase, an Ntn-hydrolase⁷ located in lysosomes, can deplete lymphoblasts from both their external and internal -L-asparagine supplies and effectively kill human B-lineage precursor leukemia cells (SUP-B15) and also T cell leukemia cells (CCRF-CEM) by apoptosis. The IC₅₀ values of glycosylasparaginase, dephosphorylated glycosylasparaginase, *Erwinia chrysanthemi* and *Escherichia coli* L-asparaginases towards SUP-B15 cells *in vitro* were 15 U/l; 16 U/l, 8 U/l and 5 U/l, respectively, showing that the cytotoxic activity of glycosylasparaginase, dephosphorylated glycosylasparaginase and bacterial L-asparginases is of the same order of magnitude. These data show that human glycosylasparaginase possesses antileukemic features similar to bacterial L-asparaginases.

Therefore, glycosylasparaginase polypeptides, including their fragments, modifications and precursors, are believed to be useful in the treatment of cancers, generally, especially in leukemias such as treatment of acute lymphoblastic leukaemia.

In its broadest aspect, this invention concerns the use of a glycosylasparaginase polypeptide having L-asparaginase activity or a precursor thereof for degradation of L-asparagine, *either in vivo* in an individual or *in vitro,* for example in a solution.

In another aspect, this invention concerns the use of a glycosylasparaginase polypeptide having L-asparaginase activity or a precursor thereof for the manufacture of a pharmaceutical composition useful in the treatment or prevention of cancer in an individual.

According to a third aspect, the invention concerns a systemic, oral, or a local non-topical pharmaceutical composition comprising an active amount of a glycosylasparaginase polypeptide having L-asparaginase activity or a precursor thereof, optionally in combination with a pharmaceutically acceptable carrier, for use in the treatment or prevention of cancer.

According to a fourth aspect, the invention concerns a pharmaceutical composition comprising an expression vector encompassing a nucleic acid sequence encoding a glycosylasparaginase polypeptide having L-asparaginase activity or a precursor thereof, said vector being capable of expressing said polypeptide or precursor in a mammalian cell, and a pharmaceutically acceptable carrier, for use in the treatment or prevention of cancer.

According to a fifth aspect, the invention concerns a glycosylasparaginase polypeptide having L-asparaginase activity or a precursor thereof for use in the treatment or prevention of cancer in an individual.

The application concerns treatment or prevention of a disease responding to degradation of L-asparagine, particularly a cancer, by administering an effective amount of a glycosylasparaginase polypeptide having L-asparaginase activity or a precursor thereof to said individual.

### BRIEF DESCRIPTION OF THE DRAWINGS

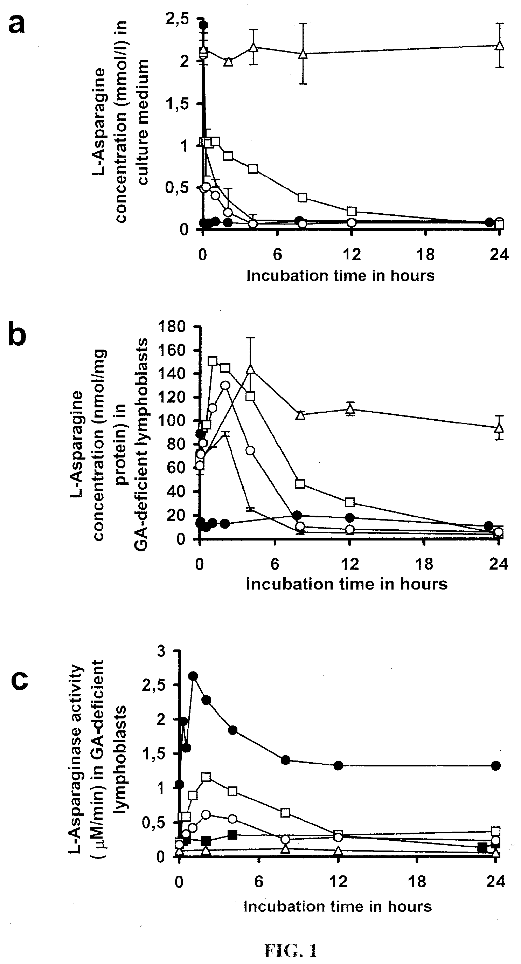

Figure 1 shows the examination of L-asparagine concentration and L-asparaginase activity in the glycosylasparaginase-deficient EBV-transformed lymphoblast culture in the presence of either GA or ErAII. L-Asn concentration in the cell culture medium (a) and inside the EBV-transformed lymphoblasts (b) after addition of GA 10 U/l (□) (single experiment), GA 40 U/l (-) (mean value of two experiments), ErAII 10 U/l (○) (single experiment), ErAII 1500 U/l (•) (single experiment) or in the absence of enzymes (Δ) (mean value of two experiments) into the culture medium. The bars represent the ± range of the values. The L-asparaginase activity inside the EBV-transformed lymphoblasts (c) after addition of GA 40 U/l (■), ErAII 40 U/l (○), ErAII 150 U/l (□), ErAII 1500 U/l (•) into the culture medium, or in the absence of enzymes (Δ). The L-asparaginase activity was determined by a spectrophotometric assay.

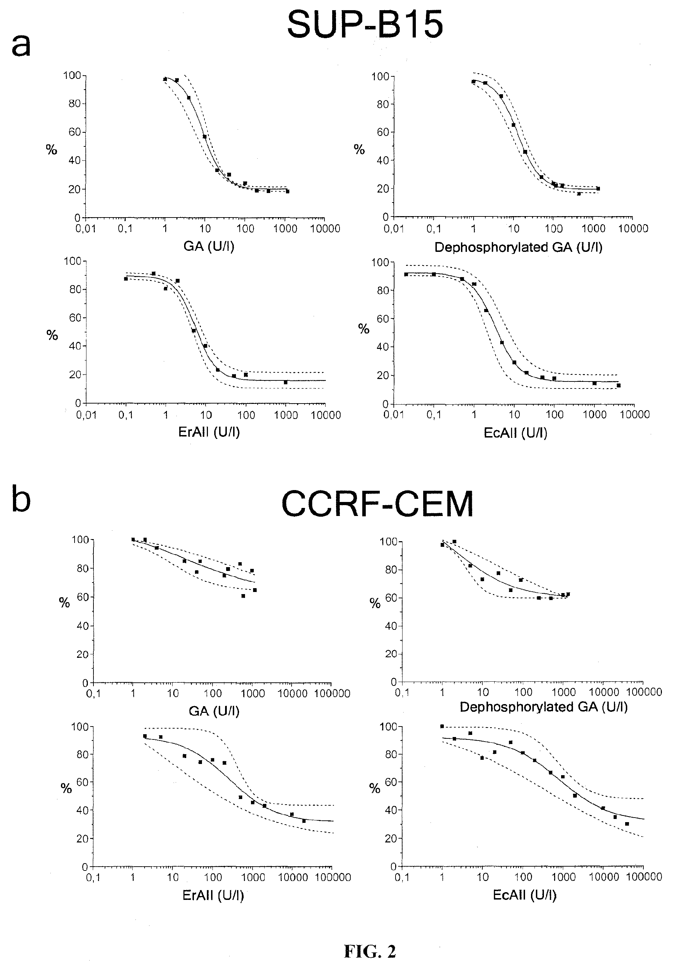

Figure 2 shows cytotoxicity of GA, dephosphorylated GA, ErAII and EcAII towards B- and T-lineage ALL cells in culture. The effect of various amounts of GA, dephosphorylated GA, ErAII and EcAII on the human B cell precursor leukemia SLTP-B15 (a) and T-lineage ALL CCRF-CEM (b) cell viability. Each data point represents the mean value of 2-5 separate experiments. The solid line represents the mean value of the cell viability and the dashed lines express ±1 SD.

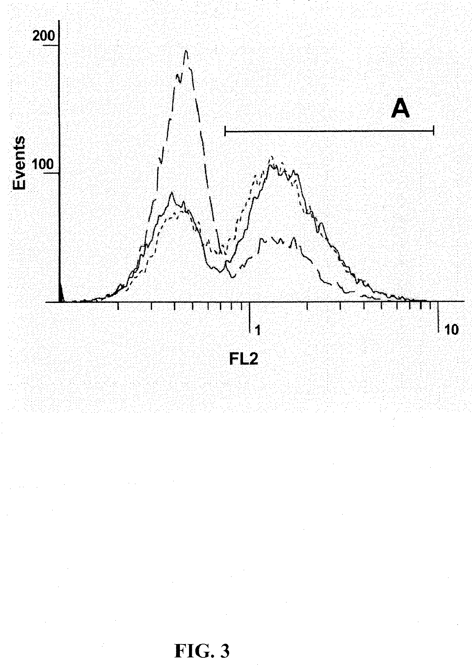

Figure 3 shows examination of apoptosis in SLTP-B15 cell population *in vitro.* The SLTP-B15 cells were incubated with 100 U/l of dephosphorylated GA for two days and stained after fixation and permeabilization with DAPI, showing all cells, and with TMR, showing only apoptotic cells of the same cell population viewed by fluorescence microscopy (pictures not shown). The apoptosis induced by 100 U/l dephosphorylated GA (solid line), 1,000,000 U/l ErAII (dotted line) or PBS (negative control, dashed line) was analyzed from fixed, permeabilized and TMR-stained SUP-B15 cells by a flow cytometer. The region labelled with A indicates the apoptotic cells.

### DETAILED DESCRIPTION OF THE INVENTION

### Definitions and preferred embodiments

As stated above, this invention concerns the use of a glycosylasparaginase polypeptide with L-asparaginase activity or a precursor thereof, for the manufacture of a pharmaceutical composition useful in treatment or prevention of a cancer in an individual.

The expression "individual" refers to a human or to an animal subject.

The term "treatment" or "treating" shall be understood to include complete curing of a disease or disorder, as well as amelioration or alleviation of said disease or disorder.

The term "prevention" shall be understood to include complete prevention, prophylaxis, as well as lowering the individual's risk of falling ill with said disease or disorder.

### The enzyme (i.e glycosylasparaginase polypeptide with L-asparaginase activity) or its precursor

The wording "L-asparaginase activity" means that the glycosylasparaginase polypeptide or its precursor is capable of catalyzing the deamination of L-asparagine to L-aspartic acid and ammonia.

The wording "precursor of a polypeptide" is understood to mean a non-active or "pro-" form of the enzyme which, after conversion, leads to the active form of the enzyme. This conversion may consist in a separation of the signal peptide and an intramolecular autoproteolysis.

The term "glycosylasparaginase polypeptide" with L-asparaginase activity shall be understood to include the following alternatives:
a) glycosylasparaginase (EC 3.5.1.26) of human, animal, microbial or vegetable origin having an L-asparaginase activity;
b) a fragment of said glycosylasparaginase, the said fragment having an L-asparaginase activity;
c) glycosylasparaginase or fragment thereof in an active form of the heterodimer or lieterotetramer type; and
d) glycosylasparaginase or a fragment thereof having undergone one or more modifications.

The expression "fragment of glycosylasparagiriase" is understood to mean any fragment characterized in that its size makes it possible to reconstitute the active site of the enzyme necessary for the activity and the specificity of glycosylasparaginase. The amino acid Threonine 206 is described as being necessary for the activity of aspartylglucosaminidase as well as the combination of its one alpha and beta chains into a heterodimer. Polypeptides having a size between 1 and 50 kD which preserve this amino acid 206 will therefore be chosen. This polypeptide fragment may be obtained by proteolysis or synthetically according to known methods.

The enzymatic polypeptide or polypeptide fragment with L-asparaginase activity according to the invention may be in the form of a precursor or in the active form of a heterodimer or heterotetramer or other polymeric form composed of heterodimers. The precursor corresponds to the non-active form of the enzyme which, after separation of the signal peptide and intramolecular autoproteolysis, leads to two subunits of 24 kDa (Alpha) and 18 kDa (Beta) whose combination in the form of a heterodimer or heterotetramer leads to the active form of the enzyme (Saarela J., 1998, The Journal of Biochemistry, Vol. 273, No. 39; 25320-25328).

The glycosylasparaginase or a fragment thereof for use in the present invention can have undergone one or more modifications. The expression "modification" is understood to mean any substitution, deletion and/or insertion of an amino acid or of a reduced number of amino acids, in particular by substitution of natural amino acids with unnatural amino acids or pseudoamino acids at positions such that the modifications do not significantly impair the biological activity of the glycosylasparaginase. The modification may also correspond to conservative substitutions, that is to say substitutions of amino acids of the same class, such as substitutions of amino acids with uncharged side chains (asparagine, glutamine, serine, threonine, tyrosine), of amino acids with basic side chains (lysine, arginine and histidine), of amino acids with acid side chains (aspartic acid and glutamic acid); of amino acids with apolar side chains (glycine, alanine, valine, leucine, isoleucine, proline, phenylalanine, methionine, tryptophan and cysteine).

In order to prolong the half-life of the glycosylasparaginase polypeptide during the therapy, the polypeptide or its precursor can be linked to polyethylene glycol or other polyols. The precursor can also be immobilized or linked to another soluble molecule, preferably to a polyol polyethylene glycol.

The glycosylasparaginase useful in this invention can originate from human or other natural sources. As examples can be mentioned the glycosylasparaginase sequences in eukaryotes, in particular the sequences of mammals (mice, humans), of yeast and of plants and their orthologues (homologous polypeptide, in a different species). As particular examples can be mentioned:

| | Swiss Prot. Acc. No. |
|---|---|
| Caenorhabditis elegans (nematode) | (Q21697) |
| Flavobacterium meningosepticum | (Q47898) |
| (bacterium) | |
| Homo sapiens (human) | (P20933) |
| Mus musculus (mouse) | (Q64191) |
| Sus scrofa (pig) | (P30918) |
| Rattus norvegicus (rat) | (P30919) |
| Spodoptera frugiperda (insect) | (O02467) |

The glycosylasparaginase polypeptide useful in the present invention, may be of natural or synthetic, in particular recombinant, origin.

The expression "natural origin" is understood to mean a polypeptide in the pure state or in solution, optionally in the presence of other protein or compound at various concentrations, which is obtained by various methods of extraction from a tissue (skin, liver and the like) of natural origin, in particular the stratum corneum of the human epidermis.

The expression "synthetic origin" is understood to mean a polypeptide in the pure state or in solution, optionally in the presence of other protein or compound at various concentrations, which is obtained chemically or by production in an organism after introduction into this organism of the elements necessary for this production.

Although human or animal glycosylasparaginase at least in principle could be derived from tissue and body fluids, this would not be a feasible source for practical use. In practice, the human glycosylasparaginase is preferably recombinant glycosylasparaginase. A method for production of recombinant human glycosylasparaginase and its purification from NIH-3T3 mouse fibroblasts is described in Mononen et al.²⁰.

### Diseases likely to respond to the treatment

It is believed that the effect of the glycosylasparaginase polypeptide for use in this invention is based on the mechanism by which cells essential for the onset of the disease are unable to synthesize asparagines. Therefore, a glycosylasparaginase polypeptide having L-asparaginase activity or a precursor thereof can be useful for any disease or disorder the treatment of which benefits from degradation of asparagines, especially L-asparagine.

Such groups of diseases are cancers, especially leukemias.

Although this invention is particularly suitable for treatment of leukemias, such as acute lymphoblastic leukemia or acute myeloid leukemia, it shall be stressed that it is not restricted thereto. Other cancers, that likely respond to this treatment are, for example multiple myeloma and solid tumors such as breast cancers, prostate cancers, and melanomas, especially multiple myeloma. Because of the much better tolerability of glycosylasparaginase, especially human glycosylasparaginase, compared to that of bacterial L-asparaginases, this drug may well be extended for use also in less severe cancerous diseases, and also for non-cancerous diseases as mentioned above.

### Formulations:

The glycosylasparaginase polypeptide can either be kept in the form of an active polypeptide or as non-active precursor in the composition up to administration.

The pharmaceutical composition may optionally comprise also an agent modulating the activity of the glycosylasparaginase polypeptide or its precursor. Such a modulating agent can be either an activator or an inhibitor.

The expression "activator" is understood to mean either a product, or a set of products, capable of stimulating the activity of the said glycosylasparaginase polypeptide or its precursor, for example of increasing the rate of the enzymatic reaction, measured by the increase in the quantity of substrates digested per unit of time during the bringing of the glycosylasparaginase polypeptide or its precursor into contact with the activator.

The expression "inhibitor" is understood to mean a product capable of inhibiting the activity of the said glycosylasparaginase polypeptide, by decreasing the rate of enzymatic reaction, measured for example by the decrease in the quantity of substrates digested per unit of time when the said product is brought into contact with the polypeptide. In particular, these products can act at the level of the active site of the enzyme or by competition in relation to binding to the substrate.

As examples of inhibitors, there may be mentioned L-asparagine which reversibly modulates glycosylasparaginase and its analogue 5-diaxo-4-oxo-L-norvaline-, which irreversibly modulates glycosylasparaginase¹⁹. There may also be mentioned N-acetylcysteine, asparagine, aspartylalanine, aspartylcyclohexylamine and p-chloromercuribenzoic acid.

The modulating agent can, depending on the kind of formulation and route of administration used, either be included in the same formulation as the glycosylasparaginase polypeptide or its precursor, or in a separate formulation. In case the drug is in the form of a non-active precursor, such a precursor may be contained in microcapsules or microgranules immersed in the composition comprising the activator.

The enzyme (i.e. the glycosylasparaginase polypeptide as defined before) or its precursor can be admixed with any carrier that is suitable for administration of the composition. For example, for parenteral administration, the enzyme or its precursor can be supplied as solution in single-dose vials for use in injections or infusions. In one alternative, the enzyme can be supplied as a freeze-dried powder for reconstitution. According to further alternatives, the enzyme or its precursor may be incorporated in oral formulations or topical formulations such as lotions, gels, creams or the like. The enzyme or its precursor may also be complexed with a lipid, packed in a liposome, incorporated in a cyclodextrin or other complexing agent, a bioresorbable polymer or other suitable carrier for controlled release administration, or encompassed in a nanoparticle or hydrogel. Glycosylasparaginase or its precursor may also be complexed to collagen or other immobilization agent to be used in grafts inside the body or in an intra- or extracorporeal perfusion device to degrade asparagine in the solution that comes to contact with the immobilized enzyme.

The expression of the enzyme or its precursor may also be achieved by gene therapy using, for example, retroviral-mediated transfer of glycosylasparaginase gene into cells of an individual. An expression vector encompassing a nucleic acid sequence encoding the glycoasparaginase polypeptide having L-asparaginase activity or its precursor can be administered to the individual. The vector can, for example, be complexed with a lipid, packed in a liposome, incorporated in a cyclodextrin or other complexing agent, a bioresorbable polymer or other suitable carrier for controlled release administration, or encompassed in a nanoparticle or hydrogel. The nucleic acid sequence can be inserted in a DNA sequence, RNA sequence or in a viral vector, which for example is based on an adenovirus, an alphavirus, adeno-associated virus, a retrovirus or a herpes virus.

The expression "local, non-epicutaneous administration" used in the composition claim below shall be understood to include any administration directed to a specific target site in the human or animal body except for administration onto the patient's skin.

The therapeutically effective amount of the enzyme or its precursor, or the expression vector, to be given to a patient in need of such treatment may depend upon a number of factors including, for example, the age and weight of the patient, the precise condition requiring treatment and its severity, and the route of administration. The precise amount will ultimately be at the discretion of the attending physician. Thus, practice of the present invention may involve any dose, combination with other therapeutically effective drugs, pharmaceutical formulation or delivery system for parenteral, topical or oral administration. The enzyme or its precursor can be administered systemically or locally. As suitable routes of administration can be mentioned intravenous, intramuscular, subcutaneous injection, oral, topical, ocular, rectal and intraperitoneal delivery and transdermal delivery systems.

For use in treatment of acute leukemia we believe that a suitable i.v. dose can be found in the range 100 to 1000 IU/kg body weight, preferably about 200 IU/kg body weight.

In treatment of cancer, it may be preferable to use the glycosylasparaginase therapy as a combination therapy with other chemotherapeutic agents such as vincristine, methotrexate, cytarabine, daunorubicin or doxorubicin, or with other cancer therapies such as radiotherapy.

The invention will be illuminated by the following non-restrictive Experimental Section.

### Experimental section

### Materials and Methods

### Enzymes

*Erwinia chrysanthemi* L-asparaginase (ErAII, Erwinase^{®}) (10,000 IU/ampoule, Porton Products) was dissolved in 1 ml of sterile phosphate buffered saline (PBS) (Reagena) to have a final concentration of 10,000,000 U/l, and *E. coli* L-asparaginase (EcAII, Elspar^{®}) (10,000 IU/ampoule, Merck) was dissolved in 5 ml of sterile PBS to a final concentration of 2,000,000 U/l. The specific activity of ErAII towards L-asparagine (Fluka) was determined by high pressure liquid chromatography (HPLC) as described²⁵. The column was Spherisorb S3 ODS2 3µm, 15 cm x 4.6 mm (Waters). Organic solvents for HPLC analysis were purchased from Rathburn Chemicals Ltd. Carboxymethyl cysteine (CmCys) and phenylisothiocyanate (PITC) were products of Sigma.

Human recombinant glycosylasparaginase (GA) was purified from NIH-3T3 mouse fibroblasts as described previously²⁰ and then dialysed into PBS with a Microcon^{®} 10 microconcentrator (Amicon) and sterile filtered. The enzyme activity of the GA preparation was measured towards L-aspartic acid β-(7-amido-4-methylcoumarin), AspAMC, (BACHEM) by SpectraFluor fluorometer (Tecan) or towards Asn with HPLC as described above. The specific activities of GA towards AspAMC and Asn were 0.25 U/mg and 0.99 U/mg, respectively. The final activity of the sterile filtered stock preparation of GA was 5880 U/l.

Dephosphorylation of GA (4.4 U) with alkaline phosphatase (AFOS) (Sigma) was carried out at 37°C for 3.5 h in the presence of 1.6 U of AFOS in 50 mM Tris-HCl, pH 8, in a total volume of 1 ml. After incubation, the dephosphorylated GA was dialysed into PBS and then sterile filtered. The final activity of the sterile dephosphorylated stock preparation was 6780 U/l.

### Cell culture experiments with glycosylasparaginase-deficient Epstein-Barr virus-transformed (EBV) lymphoblasts

Epstein Barr virus -transformed (EBV) glycosylasparaginase-deficient lymphoblasts from an aspartylglycosaminuria (AGU) patient were generated as described²⁶. The cell suspension was diluted to the density of 1 x 10⁶ cells/ml in RPMI-1640 medium (HyClone) containing 15% FBS (BioClear), 71 mg/ml streptomycin, 71 U/ml penicillin, 2 mM L-glutamine (HyClone) and 1.5 - 2.0 mM L-asparagine. After an incubation period of 15 minutes at 37°C, a 3-ml sample of the cell culture mixture was taken for the determination of the initiate value of the enzyme activity and the Asn concentration inside the cells as well as in the culture medium. Either GA or ErAII was added to the cell culture medium to the final activity of 8-47 U/l and 10-1517 U/l, respectively. The control cells were cultured in the absence of the enzymes. The cells were cultured at 37°C for 27-48 hours and 3 ml aliquots were withdrawn periodically for biochemical analysis. The samples were kept on ice until centrifugation (220 x g) for 5 minutes at 4°C. The medium was separated and stored at -20°C until assay. The pelleted cells were washed twice with phosphate buffered saline (PBS) and then lysed in 50 mM sodium-potassium phosphate buffer, pH 7.5, via three freezing-thawing cycles and sonication in an ice bath for 30 seconds. The culture medium and cell lysate samples were PITC derivatized and their Asn concentration was analyzed with HPLC²⁵. Cell lysates and culture medium were also assayed for their GA or ErAII activity with a spectrophotometric method²⁷ or a fluorometric assay²⁸ using Asn or AspAMC as the substrate, respectively. Total protein of the cell lysates and GA preparate was measured by a Protein Assay kit (BIO-RAD) according to the manufacturer's instructions.

### Cell culture experiments with human B cell precursor leukemia SUP-B15 cells and human T cell leukemia CCRF-CEM cells

### Determination of IC₅₀ values

The Cell Proliferation Kit I (MTT) (Boehringer Mannheim), which has been successfully used for the analysis of viable lymphoid and bone marrow cells^{29,30,10}, was used according to the manufacturer's instructions. Briefly, 30 µl of PBS as a negative control or various concentrations of the GA, dephosphorylated GA, ErAII or EcAII dissolved in PBS were added in duplicate into microtiter plate wells. The SUP-B15 cells (DSMZ) were suspended to McCoy's 5A medium containing 20% FBS to a final density of 2 x 10⁶ viable cells/ml and the CCRF-CEM cells (DSMZ) were suspended to RPMI-1640 cell culture medium containing 10% FBS to a final density of 1 x 10⁶ viable cells/ml, respectively. 120 µl of either SUP-B15 or CCRF-CEM cell suspension was added to the microtiter plate wells and after three (SUP-B15) or four (CCRF-CEM) days of cultivation, the wells were photographed (Olympus, SC 35, type 12, attached to an Olympus CK 2 microscope). After addition of 15 µl of sterile MTT reagent (final concentration 0.5 mg/ml) into each well, the incubation was continued for another 4 hours at 37 °C. 140 µl of the solubilization reagent was added to wells and the plate was incubated overnight at 37 °C. Finally, the absorbance of the wells at 540 nm was measured by using a Tecan SpectraFLUOR spectrophotometer (Tecan) to detect the effect of the increasing concentrations of the different enzymes on the cells.

### Detection of apoptosis

In order to detect the apoptosis at a single cell level using the TMR test (Boehringer Mannheim), which is based on TUNEL method, the SUP-B15 cells were incubated for two days in the presence of PBS or 100 U/l of GA, dephosphorylated GA, ErAII or EcAII. The cells were fixed, permeabilized and labeled according to the manufacturer's instructions. As a positive control we used a population of SUP-B15 cells, which was incubated in PBS for two days, and after fixation and permeabilization incubated with DNase I (1 mg/ml) for 10 minutes at room temperature to induce DNA strand breaks, and finally stained with TMR. Negative control cells were resuspended after fixation and permeabilization into a solution that lacked terminal transferase thus retaining the cells unlabeled in TMR staining. The apoptotic (TMR, red) cells in the total cell population (DAPI, blue, QUANTUM Appligene) were detected by an Axioplan 2 imaging microscopy (ZEISS) and Isis *in situ-*imaging system software (MetaSystems). To determine the proportion of apoptotic cells in the whole cell population, the SUP-B15 cells were incubated in the presence of either PBS, 100 U/l dephosphorylated GA or 1,000,000 U/l ErAII for two days, and after fixation and permeabilization of the cells they were stained with TMR (not with DAPI) as described above. The apoptotic cells were analyzed with Coulter EPICS XL-MCL™ Flow Cytometer (Coulter) and EXPO™ Analysis Software, (Applied Cytometry Systems). ORIGIN™ Data Analysis Software (Microcal Software) was used to draw the cytotoxicity curves.

### Results

The ability of GA and ErAII to deplete intra- and extracellular Asn reservoirs *in vitro* was studied with EBV-transformed glycosylasparaginase-deficient lymphoblasts. The depletion of the Asn concentration in the culture medium from 2.3 to 0.2 mmol/l in the presence of 40 U/l and 10 U/l of GA took 4 h and 18 h, respectively. 10 U/l of ErAII decreased the Asn level in the culture medium from 2.1 mmol/l to below 0.1 mmol/l within 4 hours. In the presence of 1500 U/l of ErAII, the Asn concentration decreased from 2.4 mmol/l to below 0.1 mmol/l within 5 minutes. As expected, the Asn level in the cell culture medium remained unchanged at the level of 2 mmol/l in the absence of either glycosylasparaginase or ErAII. **(****Fig. 1a**).

In the presence of 40 U/l GA in the cell culture medium, the Asn concentration inside the GA-deficient cells first rose from 69 up to 89 nmol/mg protein within the first 2 hours of incubation and then decreased to 6 nmol/mg protein during the next 6 hours. In the presence of 10 U/l of GA in the cell culture medium, the Asn level inside the GA-deficient lymphoblasts first rose from 69 nmol/mg protein to 150 nmol/mg protein during the first 2 hours, then fell down to 50 nmol/mg protein during the next 6 hours followed by a slow decrease to the level of 5 nmol/mg protein within the next 16 hours. In the presence of 10 U/l ErAII, the Asn concentration first rose from 62 up to 130 nmol/mg protein during the first 2-3 h of incubation and then decreased gradually to 10 nmol/mg protein within the next 5 hours. 1500 U/l of ErAII in the culture medium resulted in a rapid depletion of the intracellular Asn concentration from 89 to 14 nmol/mg protein within a minute. In the absence of either of the enzymes, the Asn levels inside the cells increased during the first 4-6 hours from 60 nmol/mg protein to 140 nmol/mg protein and then decreased slowly to around 100 nmol/mg protein during the next 20 hours of incubation **(****Fig. 1b**).

After the addition of either GA or ErAII into the culture medium, the activity of the enzymes appeared inside the cells within 5-15 minutes, reached the peak value 1-2 hours later and then decreased gradually during the next 48 hours. The enzyme activity inside the cells was dependent on its activity in the culture medium. The highest enzyme activity (2.6 µM/min) inside the cells was reached in the presence of 1500 U/l ErAII in the culture medium. 150 and 40 U/l of ErAII, and 40 U/l of GA in the culture medium resulted in the respective peak activity of 1.2, 0.6 and 0.3 µM/min of the enzymes inside the cells. In the absence of the enzymes, the L-asparaginase activity remained on the zero level both in the medium and the cells **(****Fig. 1c****).** The combined evidence shows that both GA and ErAII effectively deplete both intra- and extracellular L-asparagine reservoirs of EBV-transformed glycosylasparaginase-deficient lymphoblasts and enter the cells *in vitro.*

These results and the previous study, which demonstrated the high efficacy of glycosylasparaginase therapy in correction of the lysosomal storage of GlcNAc-Asn in the nonneuronal tissues of the GA-deficient AGU mice²¹, led us to consider GA as a potential antileukemic enzyme. Two different leukemic cell lines, human B cell precursor leukemia SUP-B15 cells and human T-lineage leukemia CCRF-CEM cells, were incubated in the presence of GA, dephosphorylated GA and two bacterial L-asparaginases, EcAII (Elspar^{®}) and ErAII (Erwinase^{®}), and the sensitivity of the cells to apoptosis was tested with the MTT assay²². The enzyme concentrations that caused 50% growth inhibition of SUP-B15 cells (IC₅₀ values) in cell culture for GA, dephosphorylated GA, ErAII and EcAII were 15 U/l, 16 U/l, 8 U/l and 5 U/l, respectively **(****Fig. 2a****).** The viability of the SUP-B15 cells after 72 hours' exposure to 1000 U/l of any of the four enzymes was approximately 20%. The results show that GA and dephosphorylated GA induce apoptosis in human B cell precursor leukemia (SUP-B15) with an efficacy comparable to bacterial L-asparaginases **(****Fig. 2a****).** T-lymphoblastoid CCRF-CEM cell line was remarkably less sensitive to all of the enzymes and the IC₅₀ -values for ErAII and EcAII were 800 U/l and 1100 U/l, respectively. After 96 hours' incubation in the presence of 100 U/l of dephosphorylated GA or native GA, the viability of the CCRF-CEM cells was 60% or 70%, respectively **(****Fig. 2b****).** A significant dose-dependent growth inhibition could also be demonstrated with each enzyme among human T cell leukemia CCRF-CEM cells, but the induction of apoptosis was less effective with GA than with ErAII and EcAII **(****Fig. 2b****).**

Our results show that both GA and dephosphorylated GA induced the death of human B cell precursor leukemia SUP-B15 cells as effectively as the clinically used L-asparaginases, ErAII and EcAII. Our finding that the CCRF-CEM cells were as sensitive to dephosphorylated GA as to native GA underlines the importance of the extracellular source of Asn for their living, since dephosphorylated GA poorly enters the cells and depletes more effectively Asn outside the cells than native GA, which enters the cells. Our experiments with EBV-transformed GA-deficient lymphoblasts showed that both extra- and intracellular L-asparagine reservoirs were destroyed by the enzymes almost simultaneously during the first 12 hours of incubation indicating that the efficacy of dephosphorylated GA in the leukemic cell cultures is due its ability to deplete the extracellular source of L-Asn. Our results are consistent with the previous studies showing the CCRF-CEM cells poorly sensitive²³ and the SUP-B 15 cells highly sensitive²⁴ to L-asparaginase therapy.

The late event of apoptosis, DNA fragmentation, was examined in SUP-B 15 cells after two days of their incubation in the presence of 100 U/l dephosphorylated GA. The evaluation of the cells under fluorescence microscopy and their analysis with flow cytometer revealed that most of the cells incubated with dephosphorylated GA were apoptotic. The proportion of the apoptotic cells in the SUP-B 15 cell population after an incubation in the presence of either PBS, PBS + DNase, 100 U/l dephosphorylated GA or 1,000,000 U/l ErAII was 28%, 90%, 65% and 69%, respectively **(****Fig 3****).**

The combined data clearly show that the antileukemic characteristics of glycosylasparaginase towards human B- and T-lineage leukemia cells are comparable to those of bacterial L-asparaginases. This along with the fact that glycosylasparaginase is a human protein without any L-glutaminase activity¹⁸ suggests that it might be less immunogenic to the human body than bacterial L-asparaginases and might have potential as an anti-cancer drug to induce apoptosis in such tumour or leukaemia cells, which are dependent on the external supply of L-asparagine.

It will be appreciated that the present invention can be incorporated in the form of a variety of embodiments, only a few of which are disclosed herein. Thus, the described embodiments are illustrative and should not be construed as restrictive.

### REFERENCES

1. Keating, M., Holmes, R., Lerner, S. & Ho, D. L-asparaginase and PEG asparaginase - past, present, and future. Leukemia and Lymphoma 10, 153-157 (1993).
2. Müller, H. & Boos, J. Use of L-asparaginase in childhood ALL. Crit. Rev. Oncol. 28, 97-113 (1998).
3. Capizzi, R. et al. L-asparaginase: clinical, biochemical, pharmacological and immunological studies. Ann. Intern. Med. 74, 893-901 (1971).
4. Boos, J. Pharmacokinetics and drug monitoring of L-asparaginase treatment. Int. J. Clin. Pharmacol. Ther. 35, 96-98 (1997).
5. Oettgen, H. et al. Toxicity of E. coli L-asparaginase in man. Cancer 25, 253-277 (1970).
6. Billett, A., Carls, A., Gelber, R. & Sallan, S. Allergic reactions to Erwinia asparaginase in children with acute lymphoblastic leukemia who had previous allergic reactions to Escherichia coli asparaginase. Cancer 70, 201-206 (1992).
7. Brannigan JA, Dodson G, Duggelby HJ et al: A protein catalytic framework with an N-terminal nucleophile is capable of self-activation. Nature 378, 416-419 (1995).
8. Okada, S. et al. In vitro efficacy of L-asparaginase in childhood acute myeloid leukaemia. Br. J. Haematol. 123, 802-809 (2003).
9. Story, M., Voehringer, D., Stephens, L. & Meyn, R. L-asparaginase kills lymphoma cells by apoptosis. Cancer Chemother. Pharmacol. 32, 129-133 (1992).
10. Holleman, A., den Boer, M., Kazemier, K., Janka-Schaub, G. & Pieters, R. Resistance to different classes of drugs is associated with impaired apoptosis in childhood acute lymphoblastic leukemia. Blood 102, 4541-4546 (2003).
11. Howard, J. & Carpenter, F. L-Asparaginase from Erwinia Carotovora. J. Biol. Chem. 247, 1020-1030 (1972).
12. Ollenschläger, G. et al. Asparaginase-induced derangements of glutamine metabolism: the pathogenetic basis for some drug-related side-effects. Eur. J. Clin. Invest. 18, 512-516 (1988).
13. Wang, B. et al. ELISA to evaluate plasma anti-asparaginase IgG concentrations in patients with acute lymphoblastic leukemia. J. Immunol. Methods 239, 75-83 (2000).
14. Woo, M. et al. Anti-asparaginase antibodies following E. coli asparaginase therapy in pediatric acute lymphoblastic leukemia. Leukemia 12, 1527-1533 (1998).
15. Wang, B. et al. Evaluation of immunologic crossreaction of antiasparaginase antibodies in acute lymphoblastic leukemia (ALL) and lymphoma patients. Leukemia 17, 1583-1588 (2003).
16. Graham, M. Pegaspargase: a review of clinical studies. Adv. Drug Deliv. Rev. 55, 1293-1302 (2003).
17. Makino M, Kojima T, Yamashina I. Enzymatic cleavage of glycopeptides. Biochem. Biophys. Res. Comm. 24, 961-966 (1966).
18. Kaartinen, V., Mononen, T., Laatikainen, R. & Mononen, I. Substrate specificity and reaction mechanism of human glycosylasparaginase. The N-glycosidic linkage of various glycoasparagines is cleaved through a reaction mechanism similar to L-asparaginase. J Biol Chem 267, 6855-6858 (1992).
19. Noronkoski, T., Stoineva, I., Petkov, D. & Mononen, I. Recombinant human glycosylasparaginase catalyzes hydrolysis of L-asparagine. FEBS Lett. 412, 149-152 (1997).
20. Mononen, I. et al. Recombinant glycosylasparaginase and in vitro correction of aspartylglycosaminuria. The FASEB J 9, 428-433 (1995).
21. Dunder, U. et al. Enzyme replacement therapy in a mouse model of aspartylglucosaminuria. The FASEB J. 14, 361-367 (2000).
22. Hongo, T., Yajima, S., Sakurai, M., Horikoshi, Y. & Hanada, R. In vitro drug sensitivity testing can predict induction failure and early relapse of childhood acute lymphoblastic leukemia. Blood 89, 2595-2965 (1997).
23. Estlin, E., Ronghe, M., Burke, G. & Yule, S. The clinical and cellular pharmacology of vincristine, corticosteroids, L-asparaginase, anthracyclines and cyclophosphamide in relation to childhood acute lymphoblastic leukaemia. Br. J. Haematol. 110, 780-790 (2000).
24. Fine, B., Kaspers, G., Ho, M., Loonen, A. & Boxer, L. A genome-wide view of the in vitro response to L-asparaginase in acute lymphoblastic leukemia. Cancer Res. 65, 291-299 (2005).
25. Kaartinen, V. & Mononen, I. Assay of aspartylglycosylaminase by high-performance liquid chromatography. Anal. Biochem. 190, 98-101 (1990).
26. Volkman, D., Buescher, E., Gallin, J. & Fauci, A. B cell lines as models for inherited phagocytic diseases: abnormal superoxide generation in chronic granulomatous disease and giant granules in Chediak-Higashi syndrome. The Journal of Immunology 133, 3006-3009 (1984).
27. Tarentino, A. & Maley, F. The purification and properties of a β-aspartyl N-acetylglucosylamine aminohydrolase from hen oviduct. Arch. Biochem. Biophys. 130, 295-303 (1969).
28. Mononen, I., Kaartinen, V. & Williams, J. A fluorometric assay for glycosylasparaginase activity and detection of aspartylglycosaminuria. Anal. Biochem. 208, 372-374 (1993).
29. Ramakers-van Woerden, N. et al. In vitro drug-resistance profile in infant acute lymphoblastic leukemia in relation to age, MLL rearrangements and immunophenotype. Leukemia 18, 521-529 (2004).
30. Pieters, R. et al. Relation between age, immunophenotype and in vitro drug resistance in 395 children with acute lymphoblastic leukemia - implications for treatment of infants. Leukemia 12, 1344-1348 (1998).
31. Ando, M. et al. Selective apoptosis of natural killer-cell tumours by L-asparaginase. British Journal of Haematology, 130, 860-868.

## Claims

1. A glycosylasparaginase polypeptide having L-asparaginase activity or a precursor thereof for use in the treatment or prevention of cancer in an individual.

2. The glycosylasparaginase polypeptide according to claim 1, wherein said glycosylasparaginase polypeptide is selected from the group consisting of
a) glycosylasparaginase (EC 3.5.1.26) of human, animal, microbial or vegetable origin having an L-asparaginase activity;
b) a fragment of said glycosylasparaginase, the said fragment having an L-asparaginase activity;
c) glycosylasparaginase or fragment thereof in an active form of the heterodimer, heterotetramer or a larger polymer type composed of heterodimers; and
d) glycosylasparaginase or a fragment thereof having undergone one or more modifications.

3. The glycosylasparaginase polypeptide according to claim 1 or 2, wherein said glycosylasparaginase polypeptide or its precursor is human glycosylasparaginase or its precursor.

4. The glycosylasparaginase polypeptide according any of the claims 1-3, wherein said glycosylasparaginase polypeptide or its precursor is of synthetic origin, preferably recombinant glycosylasparaginase.

5. The glycosylasparaginase polypeptide according to any of the foregoing claims, wherein said glycosylasparaginase polypeptide or its precursor is linked to a polyol, preferably polyethylene glycol, or wherein its precursor is immobilized or linked to another soluble molecule, preferably to a polyol polyethylene glycol.

6. The glycosylasparaginase polypeptide according to any of the foregoing claims, wherein the disease is a cancer, such as a solid tumor or multiple myeloma or a leukemia, particularly acute lymphoblastic leukemia or acute myeloid leukaemia.

7. The glycosylasparaginase polypeptide according to any of the foregoing claims wherein said polypeptide is administered parenterally, especially intravenously or subcutaneously, or orally or topically.

8. A pharmaceutical composition for parenteral, oral or local, non-epicutaneous administration comprising an active amount of a glycosylasparaginase polypeptide having L-asparaginase activity or a precursor thereof, optionally in combination with a pharmaceutically acceptable carrier, for use in the treatment or prevention of cancer.

9. The composition according to claim 8 wherein
i) the carrier is a liquid and the pharmaceutical composition is a solution, or
ii) said composition is a glycosylasparaginase polypeptide or precursor thereof in the form of a freeze-dried powder.

10. A pharmaceutical composition comprising an expression vector encompassing a nucleic acid sequence encoding a glycosylasparaginase polypeptide having L-asparaginase activity or a precursor thereof, said vector being capable of expressing said polypeptide or its precursor in a mammalian cell, and a pharmaceutically acceptable carrier, for use in the treatment or prevention of cancer.

## Patentansprüche

1. Glycosylasparaginase-Polypeptid, das L-Asparaginaseaktivität hat, oder ein Vorläufer davon zur Verwendung bei der Behandlung oder Prävention von Krebs bei einem Individuum.

2. Glycosylasparaginase-Polypeptid gemäß Anspruch 1, wobei das Glycosylasparaginase-Polypeptid ausgewählt ist aus der Gruppe, bestehend aus
a) Glycosylasparaginase (EC 3.5.1.26) menschlicher, tierischer, mikrobieller oder pflanzlicher Herkunft, die L-Asparaginaseaktivität hat;
b) einem Fragment der genannten Glycosylasparaginase, wobei das Fragment L-Asparaginaseaktivität hat;
c) Glycosylasparaginase oder Fragment davon in einer aktiven Form des Heterodimer-, Heterotetramer- oder größeren Polymer-Typs, bestehend aus Heterodimeren, und
d) Glycosylasparaginase oder einem Fragment davon, die/das eine oder mehrere Modifikation(en) durchgemacht hat.

3. Glycosylasparaginase-Polypeptid gemäß Anspruch 1 oder 2, wobei das Glycosylasparaginase-Polypeptid oder sein Vorläufer humane Glycosylasparaginase oder ihr Vorläufer ist.

4. Glycosylasparaginase-Polypeptid gemäß einem der Ansprüche 1 bis 3, wobei das Glycosylasparaginase-Polypeptid oder sein Vorläufer synthetischer Herkunft ist, vorzugsweise rekombinante Glycosylasparaginase ist.

5. Glycosylasparaginase-Polypeptid gemäß einem der vorangehenden Ansprüche, wobei das Glycosylasparaginase-Polypeptid oder sein Vorläufer mit einem Polyol, vorzugsweise Polyethylenglykol, verknüpft ist oder wobei sein Vorläufer an einem anderen löslichen Molekül, vorzugsweise an einem Polyolpolyethylenglykol immobilisiert oder damit verknüpft ist.

6. Glycosylasparaginase-Polypeptid gemäß einem der vorangehenden Ansprüche, wobei die Erkrankung Krebs, zum Beispiel ein solider Tumor oder multiples Myelom oder Leukämie, insbesondere akute lymphoblastische Leukämie oder akute myeloische Leukämie, ist.

7. Glycosylasparaginase-Polypeptid gemäß einem der vorangehenden Ansprüche, wobei das Polypeptid parenteral, speziell intravenös oder subkutan, oder oral oder topisch verabreicht wird.

8. Pharmazeutische Zusammensetzung zur parenteralen, oralen oder lokalen, nicht-epikutanen Verabreichung, umfassend eine aktive Menge eines Glycosylasparaginase-Polypeptids, das L-Asparaginaseaktivität hat, oder eines Vorläufers davon, gegebenenfalls in Kombination mit einem pharmazeutisch annehmbaren Träger, zur Verwendung bei der Behandlung oder Prävention von Krebs.

9. Zusammensetzung gemäß Anspruch 8, wobei
i) der Träger eine Flüssigkeit ist und die pharmazeutische Zusammensetzung eine Lösung ist oder
ii) die Zusammensetzung ein Glycosylasparaginase-Polypeptid oder ein Vorläufer davon in Form eines gefriergetrockneten Pulvers ist.

10. Pharmazeutische Zusammensetzung, umfassend einen Expressionsvektor, der eine Nucleinsäuresequenz umfasst, die ein Glycosylasparaginase-Polypeptid, das L-Asparaginaseaktivität hat, oder einen Vorläufer davon codiert, wobei der Vektor in der Lage ist, das Polypeptid oder seinen Vorläufer in einer Säugetierzelle zu exprimieren, und einen pharmazeutisch annehmbaren Träger, zur Verwendung bei der Behandlung oder Prävention von Krebs.

## Revendications

1. Polypeptide glycosylasparaginase ayant une activité de L-asparaginase ou précurseur de celui-ci pour une utilisation dans le traitement ou la prévention du cancer chez un individu.

2. Polypeptide glycosylasparaginase selon la revendication 1, dans lequel ledit polypeptide glycosylasparaginase est choisi dans le groupe consistant en
a) la glycosylasparaginase (EC 3.5.1.26) d'origine humaine, animale, microbienne ou végétale ayant une activité de L-asparaginase ;
b) un fragment de ladite glycosylasparaginase, ledit fragment ayant une activité de L-asparaginase ;
c) la glycosylasparaginase ou un fragment de celle-ci sous une forme active de l'hétérodimère, d'hétérotétramère ou d'un type de polymère plus grand composé d'hétérodimères ; et
d) la glycosylasparaginase ou un fragment de celle-ci ayant subi une ou plusieurs modifications.

3. Polypeptide glycosylasparaginase selon la revendication 1 ou 2, dans lequel ledit polypeptide glycosylasparaginase ou son précurseur est la glycosylasparaginase humaine ou son précurseur.

4. Polypeptide glycosylasparaginase selon l'une quelconque des revendications 1 à 3, dans lequel ledit polypeptide glycosylasparaginase ou son précurseur est d'origine synthétique, de préférence une glycosylasparaginase recombinante.

5. Polypeptide glycosylasparaginase selon l'une quelconque des revendications précédentes, dans lequel ledit polypeptide glycosylasparaginase ou son précurseur est lié à un polyol, de préférence le poly(éthylène glycol), ou dans lequel son précurseur est immobilisé ou lié à une autre molécule soluble, de préférence un polyol poly(éthylène glycol).

6. Polypeptide glycosylasparaginase selon l'une quelconque des revendications précédentes, dans lequel la maladie est un cancer, tel qu'une tumeur solide ou un myélome multiple ou une leucémie, en particulier une leucémie lymphoblastique aiguë ou une leucémie myéloïde aiguë.

7. Polypeptide glycosylasparaginase selon l'une quelconque des revendications précédentes, dans lequel ledit polypeptide est administré par voie parentérale, en particulier par voie intraveineuse ou sous-cutanée, ou par voie orale ou topique.

8. Composition pharmaceutique pour une administration par voie parentérale, orale ou locale, non épicutanée comprenant une quantité active d'un polypeptide glycosylasparaginase ayant une activité de L-asparaginase ou un précurseur de celui-ci, facultativement en combinaison avec un vecteur pharmaceutiquement acceptable, à utiliser dans le traitement ou la prévention du cancer.

9. Composition selon la revendication 8, dans laquelle
i) le vecteur est un liquide et la composition pharmaceutique est une solution, ou
ii) ladite composition est un polypeptide glycosylasparaginase ou un précurseur de celui-ci sous la forme d'une poudre lyophilisée.

10. Composition pharmaceutique comprenant un vecteur d'expression englobant une séquence d'acide nucléique codant pour un polypeptide glycosylasparaginase ayant une activité de L-asparaginase ou un précurseur de celui-ci, ledit vecteur étant capable d'exprimer ledit polypeptide ou son précurseur dans une cellule de mammifère, et un vecteur pharmaceutiquement acceptable, pour une utilisation dans le traitement ou la prévention du cancer.
